# EUROPÄISCHE PATENTSCHRIFT

(11) **EP 2 869 854 B1**
(45) Veröffentlichungstag und Bekanntmachung des Hinweises auf die Patenterteilung: **10.04.2019**
(21) Anmeldenummer: 13737554.9
(22) Anmeldetag: 19.06.2013
(51) Int. Cl.: A61L 26/00

(54) **ZUSAMMENSETZUNG, ENTHALTEND ALS WIRKSTOFF ECTOIN ODER HYDROXYECTOIN ZUR FÖRDERUNG DER WIEDERHERSTELLUNG VON VERLETZTEM KÖRPERGEWEBE**
COMPOSITION CONTAINING ECOTINE OR HYDROXYECOTINE AS AN ACTIVE SUBSTANCES FOR PROMOTING THE REGENERATION OF INJURED BODY TISSUE
COMPOSITION CONTENANT, EN TANT QUE PRINCIPE ACTIF, DE L'ECTOÏNE OU DE L'HYDROXYECTOÏNE POUR LA RECONSTRUCTION DE TISSUS CORPORELS LÉSÉS

(30) Priorität: 09.07.2012 DE 102012013482
(43) Veröffentlichungstag der Anmeldung: 13.05.2015
(60) Teilanmeldung: 16196808.6
(73) Patentinhaber: bitop AG, 58453 Witten (DE)
(72) Erfinder: BILSTEIN, Andreas, 50129 Bergheim (DE); SCHERNER, Olaf, 45134 Essen (DE); LENTZEN, Georg, 46483 Wesel (DE)
(74) Vertreter: Schöneborn, Holger
(86) Internationale Anmeldenummer: PCT/EP2013/062708
(87) Internationale Veröffentlichungsnummer: WO 2014/009118

(56) Entgegenhaltungen:
- EP-A1- 2 570 124
- DE-A1- 10 006 578
- DE-A1- 19 540 749
- DE-A1-102007 052 380
- DE-A1-102008 039 231
- DATABASE WPI Week 200950 Thomson Scientific, London, GB; AN 2009-L73819 XP002761611, & JP 2009 161564 A (TOKIWA YAKUHIN KOGYO KK) 23. Juli 2009 (2009-07-23)
- DATABASE WPI Week 200501 Thomson Scientific, London, GB; AN 2005-002847 XP002761612, & JP 2004 331579 A (NOEVIR KK) 25. November 2004 (2004-11-25)
- DATABASE CA [Online] CHEMICAL ABSTRACTS SERVICE, COLUMBUS, OHIO, US; LI, BAOQIU ET AL: "Application of tetrahydropyrimidine and derivative (6S)-2-methyl-1,4,5,6-tetrahydropyrimidine -6-carboxylic acid in treating skin injury", XP002711556, gefunden im STN Database accession no. 2011:1517753 & CN 102 247 374 A (SHANDONG HONGLI LABORATORY ANIMAL EXPERIMENT CO., LTD., PEOP. REP. CHI) 23. November 2011 (2011-11-23)

## Beschreibung

Die Erfindung betrifft eine Zusammensetzung, mit der die Wiederherstellung von verletzter Schleimhaut gefördert werden kann.

Verletzungen von Körpergewebe können auf unterschiedliche Art und Weise zustande kommen. Insbesondere können Gewebe durch äußere Einflüsse, d. h. auf traumatische Weise entstehen. Allgemein werden derartige Gewebeverletzungen, insbesondere im Bereich der Haut oder Schleimhaut, auch als Wunde bezeichnet. Neben Gewebeverletzungen, die auf äußere Einflüsse zurückzuführen sind, sind auch nicht traumatische Verletzungen als Ulcus (Geschwür) bekannt.

Für gewöhnlich beginnt die Wiederherstellung von geschädigtem Körpergewebe auf natürliche Weise bereits kurze Zeit nach der Verletzung. Im Fall einer Wunde setzt sehr bald die Blutgerinnung ein, so dass das zerstörte Blutgefäß durch einen Blutpfropfen (Gerinnsel) verschlossen wird. In der sich anschließenden Exsudationsphase tritt Wundsekret aus, wodurch Fremdkörper und Keime aus der Wunde herausbefördert werden. Das Immunsystem sorgt für die Abtötung von Bakterien.

In einer sich anschließenden Proliferationsphase entsteht neues Bindegewebe, sodass der durch die Wunde herbeigeführte Defekt ausgefüllt wird. Schließlich wird in einer Regenerationsphase die Wunde durch Überwachsen mit Epithelzellen verschlossen, die von intaktem Epithelgewebe im Bereich der Wundränder ausgehen.

Während die natürliche Wundheilung im Regelfall und bei nicht zu großen Wunden relativ problemlos verläuft, kann es bei großflächigen Wunden unter anderem aufgrund der starken Exsudatbildung zu Komplikationen kommen. Gleiches gilt für verschiedene Ulcera. Besonders problematisch sind darüber hinaus chronische Wunden, die auf eine dauerhafte Druckbelastung zurückzuführen sind (Dekubitus) oder auch eine verzögerte Wundheilung infolge von Diabetes mellitus. Letzterer führt bei manchen Patienten zum sog. diabetischen Fußsyndrom, das für 2/3 aller Amputationen in Deutschland verantwortlich ist.

In CN 102 247 374 A wird Ectoin als heilungsfördendes Mittel für Wunden aufgetragen

Es stellt sich somit die Aufgabe, ein Mittel zur Verfügung zu stellen, mit dem die Wiederherstellung von verletzter Schleimhaut unterstützt und gefördert werden kann.

Diese Aufgabe wird erfindungsgemäß gelöst durch eine Zusammensetzung, enthaltend als Wirkstoff Ectoin, Hydroxyectoin und/oder Salze, Ester oder Amide dieser Verbindungen zur Förderung der Wiederherstellung von verletztem Körpergewebe, wobei das verletzte Körpergewebe eine Schleimhaut ist.

Bei Ectoin und Hydroxyectoin handelt es sich um Tetrahydropyrimidinderivate, die unter Stressbedingungen von extremophilen, insbesondere halophilen Mikroorganismen synthetisiert werden. Für Ectoin und Hydroxyectoin wurden bislang verschiedene Verwendungen beschrieben, beispielsweise als Moisturizer, zur Behandlung des Vascular Leak Syndroms (VLS) (DE 10 2006 056 766 A1) oder zur Behandlung von Neurodermitis (DE 103 30 243 A1). Aus der DE 100 06 578 A1 ist die Verwendung von Ectoin und seinen Derivaten zum Schutz von Biopolymeren vor dem Abbau durch degradierende Enzyme wie Proteasen, Nukleasen oder Lipasen bekannt.

Die systematische Bezeichnung für Ectoin lautet 2-Methyl-1,4,5,6-tetrahydropyrimidin-4-carbonsäure, für Hydroxyectoin 5-Hydroxy-2-methyl-1,4,5,6-tetrahydropyrimidin-4-carbonsäure.

Die Struktur des natürlichen L-Ectoins ((S)-2-Methyl-1,4,5,6-tetrahydropyrimidin-4-carbonsäure) ist im Folgenden dargestellt:

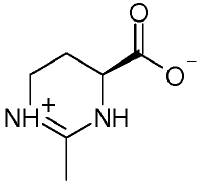

Die Struktur des natürlichen Hydroxyectoins ((4S,5S)-5-Hydroxy-2-methyl-1,4,5,6-tetrahydropyrimidin-4-carbonsäure) wird im Folgenden wiedergegeben:

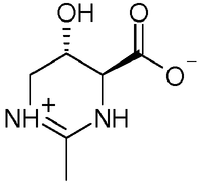

Die Verwendung der angegebenen Stereoisomere ist bevorzugt, jedoch nicht obligatorisch, d.h. auch die Verwendung anderer Stereoisomere bzw. des Racemats ist möglich.

Bei dem Körpergewebe, dessen Wiederherstellung erfindungsgemäß durch die Ectoin oder Hydroxyectoin enthaltenden Zusammensetzungen gefördert werden kann, handelt es sich um Schleimhaut.

Im Falle von geschädigten Schleimhäuten spricht man auch von Mukositis, deren Behandlung unter die Förderung der Wiederherstellung von verletztem Körpergewebe gemäß Erfindung fällt. Eine Mukositis kann unterschiedliche Ursachen haben. Da Schleimhautzellen eine hohe Regenerationsrate aufweisen, tritt eine Mukositis beispielsweise häufig als Nebenwirkung im Rahmen der Krebsbehandlung durch Chemo- oder Strahlentherapie auf. Darüber hinaus sorgt ein geschwächtes Immunsystem, beispielsweise bei immunsupprimierten Patienten, für eine Zunahme von Infektionen, die ihrerseits zu Entzündungen der Schleimhaut führen können.

Insbesondere können die Mundschleimhäute sowie die Schleimhäute des Verdauungssystems (Magen, Darm) betroffen sein.

Die erfindungsgemäße Zusammensetzung ist insbesondere für die Verabreichung auf das verletzte Körpergewebe vorgesehen, d. h. die Anwendung erfolgt insbesondere lokal bzw. topisch. Entsprechend kann die Zusammensetzung als Salbe, Creme, Lotion, Milch, Flüssigkeit, Emulsion, Mikroemulsion, Spray, Suspension, Paste, Pulver oder als sonstiger Feststoff vorliegen.

Die Zusammensetzung kann übliche Hilfsstoffe enthalten, z. B. Trägermittel, Konservierungsmittel, Bakterizide, Lösungsvermittler, Vitamine, Stabilisatoren, Substanzen zum Verhindern des Schäumens, Verdickungsmittel, Farbstoffe, oberflächenaktive Substanzen, Emulgatoren, feuchthaltende Substanzen u.ä..

Salben, Pasten, Cremes und Gele können die üblichen Trägerstoffe enthalten, z. B. tierische und pflanzliche Fette, Wachse, Paraffine, Stärke, Traganth, Zellulosederivate, Polyethylenglykole, Silikone, Bentonite, Kieselsäure, Talkum und Zinkoxid oder Gemische dieser Stoffe.

Die Zusammensetzungen können weitere Wirkstoffe enthalten, es ist jedoch insbesondere auch möglich und für die Förderung der Wiederherstellung des Körpergewebes hinreichend, Zusammensetzungen zu verwenden, die lediglich Ectoin und/oder Hydroxyectoin bzw. entsprechende Salze, Ester oder Amide als Wirkstoff enthalten.

Möglich ist beispielsweise die Kombination von Ectoin, Hydroxyectoin bzw. entsprechenden Derivaten mit einem oder mehreren Wirkstoffen ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

Die Konzentration an Ectoin/Hydroxyectoin und/oder entsprechenden Salzen, Estern oder Amiden kann insbesondere in einem Bereich zwischen 0,01 und 50 Gew.-%, insbesondere zwischen 0,5 und 20 Gew. % und besonders bevorzugt zwischen 1 und 10 Gew.-% liegen.

### Beispiel 1: Re-Epithelialisierung bei Inkubation mit Ectoin/Hydroxyectoin

In einem konfluenten Zellrasen von ARPE-19 Zellen (adhärente retinale Pigmentepithelzellen) wurden nach 24 Stunden Inkubation mit 0, 0,1, 0,5, 1, 2,5 und 5 mM Ectoin/Hydroxyectoin in serumfreiem und serumhaltigem Medium definierte Bereiche an Zellen entfernt ("Scratchassay"). An markierten Stellen wurde die Breite des beigebrachten Spalts im Mikroskop bestimmt. Jeweils 24 und 48 Stunden danach erfolgte eine Fotodokumentation und die Spaltbreite wurde an den entsprechenden Stellen erneut bestimmt. Für jede Konzentration wurden 6 Tests durchgeführt.

Die größten Zuwachsraten der Re-Epithelialisierung waren in den ersten beiden Tagen beobachtbar. Dabei nahm die Spaltbreite in mit Ectoin/Hydroxyectoin behandelten Zellkulturen rascher ab. Hydroxyectoin war für die Re-Epithelialisierung etwas effektiver als Ectoin. Serum förderte den Spaltschluss ganz allgemein, die positiven Ergebnisse eines Zusatzes von Ectoin/Hydroxyectoin waren besonders in den serumfreien Kulturen messbar.

Der Mittelwert der Spaltbreiten nach 24 h in der nicht mit Ectoin behandelten Probe wurde auf 100 % gesetzt. Es konnten die folgenden prozentualen Spaltbreiten beobachtet werden:

| **c(Ectoin)/mM** | **Spaltbreite in %** |
|---|---|
| 0 | 100 |
| 0,1 | 100,687 |
| 0,5 | 96,434 |
| 1 | 91,632 |
| 2,5 | 93,012 |
| 5 | 88,418 |

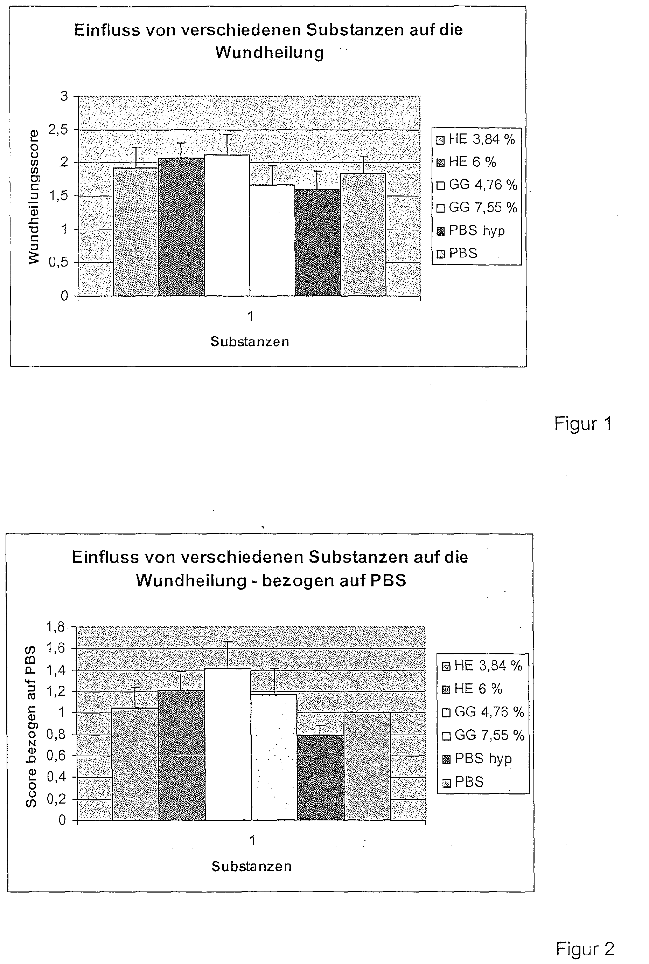

## Patentansprüche

1. Zusammensetzung, enthaltend als Wirkstoff Ectoin, Hydroxyectoin und/oder Salze, Ester oder Amide dieser Verbindungen zur Verwendung in der Förderung der Wiederherstellung von verletztem Körpergewebe, **dadurch gekennzeichnet, dass** das Körpergewebe eine Schleimhaut ist.

2. Zusammensetzung zur Verwendung nach Anspruch 1, **dadurch gekennzeichnet, dass** die Verletzung des Körpergewebes auf einem Ulcus oder einer Mukositis beruht.

3. Zusammensetzung zur Verwendung nach Anspruch 2, **dadurch gekennzeichnet, dass** die Mukositis als Nebenwirkung einer Chemo- oder Strahlentherapie oder bei immunsupprimierten Patienten auftritt.

4. Zusammensetzung zur Verwendung nach Anspruch 2 oder 3, **dadurch gekennzeichnet, dass** die Mukositis eine Mukositis der Mundschleimhaut oder der Schleimhäute des Verdauungssystems, insbesondere des Magens oder Darms ist.

5. Zusammensetzung zur Verwendung nach einem der Ansprüche 1 bis 4, **dadurch gekennzeichnet, dass** die Zusammensetzung einen oder mehrere weitere Wirkstoffe enthält, ausgewählt aus: Dexpanthenol oder Derivate, Arnica montana-Extrakt (Arnica), Capsaicin, Capsicum-Extrakt, Hypericum perforatum-Extrakt (Johanniskraut), Cardiospermum halicacabum (Ballonrebe, Herzsame), Hamamelis virginiana-Extrakt (Zaubernuss), Tocopherol, Allantoin, Bisabolol, Kakao-Extrakt, Silber, Nanosilber, Mikrosilber, amorphes Silber, Silber-Salze, Zink, Zinkoxid, Calendula officinalis-Extrakt (Ringelblume), Honig und Honigextrakte, Propolis, Melilotus officinalis-Extrakt, Beinwell-Extrakt (Symphytum), Echium vulgare-Extrakt, Cumin, Angelica sinensis-Extrakt, ferulic acid (Ferularäure), hyaluronic acid (Hyaluronsäure), Aloe Vera-Extrakt, Matricaria recutita (Kamille)-Extrakt, Allium cepa-Extrakt (Zwiebel), Achillea millefolium-Extrakt (Schafgarbe), Glycyrrhiza inflata-Extrakt (Süßholz), Licochalcon A, Silikon, Urea (Harnstoff), Echinacea purpurea (Sonnenhut)-Extrakt, Chichoriensäure.

## Claims

1. Composition containing as active agent ectoine, hydroxyectoine, and/or salts, esters or amides of these compounds for use in promoting the regeneration of injured body tissue, **characterized in that** the body tissue is a mucous membrane.

2. Composition for use according to claim 1, **characterized in that** the injury of the body tissue is an ulcer or mucositis.

3. Composition for use according to claim 2, **characterized in that** the mucositis occurs as a side effect of chemo- or radiotherapy or in immunosuppressed patients.

4. Composition for use according to claim 2 or 3, **characterized in that** the mucositis is a mucositis of the oral mucosa or the mucous membranes of the digestive system, in particular of the stomach or intestine.

5. Composition for use according to any of claims 1 to 4, **characterized in that** the composition contains a substance or a plurality of further substances to be selected from: dexpanthenol or derivatives, arnica montana extract (arnica), capsaicin, capsicum extract, hypericum perforatum extract (St John's wort), cardiospermum halicacabum (balloon plant), hamamelis virginiana extract (witch hazel), tocopherol, allantoin, bisabolol, cocoa extract, silver, nanosilver, microsilver, amorphous silver, salts of silver, zinc, zinc oxide, calendula officinalis extract (marigold), honey and honey extracts, propolis, melilotus officinalis extract, comfrey extract (symphytum), echium vulgare extract, cumin, angelica sinensis extract, ferulic acid, hyaluronic acid, aloe vera extract, matricaria recutita (chamomile) extract, allium cepa (onion) bulb extract, achillea millefolium extract (yarrow), glycyrrhiza inflate extract (licorice), licochalcon A, silicone, urea, echinacea purpurea (purple coneflower) extract, chicoric acid.

## Revendications

1. Composition, contenant en tant qu'agent actif de l'ectoïne, de l'hydroxyectoïne et/ou des sels, esters ou amides de ces composés, destinée à une utilisation pour favoriser la régénération d'un tissu corporel lésé, **caractérisée en ce que** le tissu corporel est une muqueuse.

2. Composition destinée à une utilisation selon la revendication 1, **caractérisée en ce que** la lésion du tissu corporel consiste en un ulcère ou une mucite.

3. Composition destinée à une utilisation selon la revendication 2, **caractérisée en ce que** la mucite se produit en tant qu'effet secondaire d'une chimio- ou radiothérapie ou chez des patients immunosupprimés.

4. Composition destinée à une utilisation selon la revendication 2 ou 3, **caractérisée en ce que** la mucite est une mucite de la muqueuse buccale ou des muqueuses du système digestif, notamment de l'estomac ou de l'intestin.

5. Composition destinée à une utilisation selon l'une quelconque des revendications 1 à 4, **caractérisée en ce que** la composition contient un ou plusieurs agents actifs supplémentaires, choisis parmi : le dexpanthénol ou ses dérivés, l'extrait d'Arnica montana (arnica), la capsaïcine, l'extrait de capsicum, l'extrait d'Hypericum perforatum (millepertuis), Cardiospermum halicacabum (coeur des Indes, pois de coeur), l'extrait d'Hamamelis virginiana (hamamélis), le tocophérol, l'allantoïne, le bisabolol, l'extrait de cacao, l'argent, le nano-argent, le micro-argent, l'argent amorphe, les sels d'argent, le zinc, l'oxyde de zinc, l'extrait de Calendula officinalis (souci), le miel et les extraits de miel, le propolis, l'extrait de Melilotus officinalis, l'extrait de consoude (symphytum), l'extrait d'Echium vulgare, le cumin, l'extrait d'Angelica sinensis, l'acide férulique, l'acide hyaluronique, l'extrait d'Aloe Vera, l'extrait de Matricaria recutita (camomille), l'extrait d'Allium cepa (oignon), l'extrait d'Achillea millefolium (achillée), l'extrait de Glycyrrhiza inflata (réglisse), la licochalcone A, la silicone, l'urée, l'extrait d'Echinacea purpurea (échinacée), l'acide chicorique.
